# EUROPÄISCHE PATENTSCHRIFT

(11) **EP 0 741 869 B1**
(45) Veröffentlichungstag und Bekanntmachung des Hinweises auf die Patenterteilung: **05.04.2000**
(21) Anmeldenummer: 95907632.4
(22) Anmeldetag: 31.01.1995
(51) Int. Cl.: G01N 33/68, C07K 7/02, G01N 33/566, G01N 33/543, A61K 39/21, C07K 16/08, G01N 33/541, G01N 33/58, C07K 1/04

(54) **SPEZIFISCHE BINDUNGSSUBSTANZEN FÜR ANTIKÖRPER UND DEREN VERWENDUNG FÜR IMMUNOASSAYS ODER VAKZINE**
SPECIFIC BINDING SUBSTANCES FOR ANTIBODIES AND THEIR USE FOR IMMUNOASSAYS OR VACCINES
SUBSTANCES DE FIXATION SPECIFIQUES POUR ANTICORPS ET LEUR UTILISATION DANS DES DOSAGES IMMUNOLOGIQUES OU DES VACCINS

(30) Priorität: 31.01.1994 DE 4402756
(43) Veröffentlichungstag der Anmeldung: 13.11.1996
(73) Patentinhaber: Roche Diagnostics GmbH, 68298 Mannheim (DE)
(72) Erfinder: SEIDEL, Christoph, D-82362 Weilheim (DE); HERRMANN, Rupert, D-82362 Weilheim (DE); HOESS, Eva, D-82319 Starnberg (DE); BATZ, Hans-Georg, D-82327 Tuzting (DE)
(74) Vertreter: Huber, Bernhard, Dipl.-Chem.
(86) Internationale Anmeldenummer: EP9500333
(87) Internationale Veröffentlichungsnummer: WO9520764

(56) Entgegenhaltungen:
- WO-A-91/13909
- WO-A-93/23031
- WO-A-94/02614
- KONTAKTE (DARMSTADT), Nr.2, 1993, DARMSTADT, DE Seiten 48 - 56 D. DORSCH ET AL. 'HIV Protease Inhibitors - a Promising New Class of AIDS Therapeutics'
- ANGEWANDTE CHEMIE. INTERNATIONAL EDITION, Bd.32, Nr.9, September 1993, WEINHEIM DE Seiten 1244 - 1267 ATHANSSIOS GIANNIS; THOMAS KOLTER 'Peptidomimetics for Receptor Ligands -Discovery, Development, and Medical Perspectives' & ANGEWANDTE CHEMIE, Bd.105, 1993, WEINHEIM DE Seite 105FF. A. GIANNIS; TH. KOLTER

## Beschreibung

Die Erfindung betrifft spezifische Bindungssubstanzen für Antikörper, deren Verwendung in Immunoassays und als Vakzin sowie ein immunologisches Bestimmungsverfahren unter Verwendung dieser spezifischen Bindungssubstanz als Bindungspartner für den Antikörper.

Von Antikörpern ist bekannt, daß sie die entsprechenden Antigene äußerst spezifisch binden. Ist ein Antikörper gegen eine Aminosaureteilsequenz (Epitop) eines Proteins gerichtet, ist bekannt, daß zu seiner Bindung auch diese Teilsequenz allein verwendet werden kann.

Die spezifische Bindung zweier Biomoleküle wurde erstmals für die Enzymsubstrat-Bindung mit dem Schlüssel-Schloß-Prinzip verglichen. Linus Pauling erkannte jedoch in den 60er Jahren, daß zwischen der Enzym-Substrat-Bindung und einer Antikorper-Antigen-Bindung ein fundamentaler Unterschied besteht. Die Bindungsstelle eines Enzym ist durchaus nicht vollkommen passend für sein Substrat. Vielmehr scheint die Bindungsstelle eines Enzyms der Struktur des Ubergangszustands einer Substratreaktion viel ähnlicher zu sein und erlaubt daher dem Substrat einen gewissen Spielraum in seiner Struktur, ohne daß das Enzym seine Bindungseigenschaften für das Substrat verliert. Dagegen wird für die Antigen-Antikörper-Bindung ein absolutes Einpassen des Antigen-Epitops in die Bindungsstelle des Antikörpers gefordert was eine raumliche Variation des Epitops bezüglich der Antikörperbindung prinzipiell ausschließt. Nur durch diese spezifische Epitop-Antikörper-Bindung kann eine Fremdsubstanz spezifisch aus dem Organismus durch Antikörper eliminiert werden.

Epitope, gegen die ein Antikörper spezifisch gerichtet ist, werden hauptsachlich für zwei Anwendungen einsiesetzt: Zum einen dienen sie - meist an einen Carrier gebunden - als Immunogene (Vakzine), die einen Organismus zur Produktion von Antikörpern veranlassen, die gegen diese Epitope oder gegen Antigene, die diese Epitope enthalten, gerichtet sind. Zum anderen werden solche Epitope in Immunoassays zum spezifischen Nachweis von Antikörpern in Körpertlussigkeiten eingesetzt, in denen sie durch Antigene, die diese Epitope enthalten, als Immunreaktion erzeugt wurden (beispielsweise nach Infektion durch einen infektiosen Organismus).

Sind die Epitope Polypeptide, so stellt sich bei deren Einsatz im Organismus als Vakzin oder bei diagnostischen Anwendungen im Kontakt mit Körperrlüssigkeiten wie Serum das Problem, daß die Polypeptide durch metabolischen Abbau, insbesondere Proteasen, wie sie in Körperrlüssigkeiten enthalten sind, zersetzt werden und ihre Funktion verlieren.

Von Polypeptiden, die zu therapeutischen Zwecken. d.h. zur Bindung an Enzyme oder an Hormonrezeptoren im Organismus eingesetzt werden, ist bekannt, daß sie vor ihrem metabolischem Abbau im Körper dadurch geschützt werden können, daß eine Modifikation der Aminosäuresequenz vorgenommen wird. Solche sogenannten Pepridmimetika als therapeutische Mittel und deren Herstellung werden u.a. in Giannis, Angewandte Chemie 105 (1993) 1303-1326; Lee. Bull Chem. Soc. Jpn 66 (1993) 2006-2010 und Dorsch et al, Kontakte (Darmstadt) 1993 (2) beschrieben. Giannis und Kolter offenbaren im genannten Artikel Peptidmimetika für Rezeptorliganden, welche strukturelle Modifikationen aufweisen. Es finden sich jedoch keine Hinweise auf immunologische Bindungssubstanzen. Dieser in den obigen Literaturstellen beschriebene Weg der Modifikation schien für Epitope zur Bindung von Antikörpern und zur Erzeugung von Antikörpern als Immunogen aufgrund der bekannten hohen Spezifität der Epitop-Antikörper-Bindung versperrt.

In der WO 91/13909 sind synthetische Polypeptide offenbart, die wenigstens eine antigene Eigenschaft des HIV Hüllproteins aufweisen und ausschließlich aus natürlichen Aminosäuren zusammensetzt sind.

Auch die WO 94/02614 (stand der Technik gemäß Art. 54 (3) EPÜ) befaßt sich mit Peptiden, welche ein HIV gp 120 Epitop nachahmen, sowie deren Verwendung als Vakzin. Auf Mimotope von anderen Viren wird nicht hingewiesen.

Zur Stabilisierung solcher Polypeptidepitope wurden kürzlich auf dem 3. Europäischen BIAcore Symposium in London 1993 Peptid-Humanserumalbumin-Fusionsprodukte vorgeschlagen (Integration of Biocore in the Discovery Department. S. Reboul et al). Durch die Kopplung der Peptidepitope an Humanserumalbumin wird allerdings der enzymatische Abbau der Polypeptide lediglich etwas verzögert, nicht aber verbinden.

Aufgabe der Erfindung war es. Bindungssubstanzen zur Verfügung zu stellen die als Epitope für Vakzine zur Erzeugung von Antikörpern und in Immunoassays zum Nachweis von Antikörpern, die gegen Epitope einer bestimmten naturlichen Aminosäuresequenz gerichtet sind, fungieren konnen und die eine veränderte metabolische Stabilität insbesondere eine veränderte Proteasestabilität oder eine veränderte Wirkungsdauer als Immunogen in einem tierischen Organismus oder als Bindunuspartner in Immunoassays mit Körperflüssigkeiten als Probenmaterial aufweisen und die trotzdem einen Antikörper, der gegen ein entsprechendes natürliches Epitop gerichtet ist, spezifischer und selektiver Weise binden können bzw. diesen Antikörper in einer Immunantwort erzeugen.

Die Aufgabe wird gelöst durch die Erfindung, wie sie in den Ansprüchen charakterisiert ist. Überraschenderweise wurde gefunden, daß trotz der äußerst hohen Spezifitat der Antigen-Antikörper-Reaktion bei einer Strukturänderung in einer naturlichen HCV-Epitop-Polypeptidsequenz durch Veränderung der Aminosäuren in der Sequenz in der erfinderischen Weise nicht die Bindung des modifizierten HCV-Epitops zu einem Antikörper, der gegen das unmodifizierte HCV-Epitop gerichtet ist, verhindert wird. Gleichzeitig zeigt das modifizierte HCV-Epitop im Vergleich zum unmodifizierten HCV-Epitop aber veränderte metabolische Eigenschaften im Kontakt mit Körperflüssigkeiten oder in einem tierischen Metabolismus, z.B. eine veränderte Stabilität gegenüber körpereigenen Proteasen.

Unter Veränderung der Aminosäuren der Aminosauresequenz des natürlichen HCV-Epitops werden erfindungsgemäß folgende Möglichkeiten verstanden:
a)
   Ersatz der -CO-NH-Gruppe mindestens einer Peptidbindung durch eine davon verschiedene zwei- oder drei-atomige Brücke ("back-bone Modifizierung").
   Unter zwei-atomiger Brücke wird bevorzugt eine -NH-CO-, -CH2-CH2-, -CH=CH, -CH2-NH-, -NH-CH2-, -O-CH2-, -CH2-O-, -CH2-S, oder -S-CH2-Gruppe verstanden. Unter drei-atomiger Brücke wird bevorzugt eine -CH2-CH2-CH2-Gruppe verstanden, in der eine -CH2-Gruppe auch durch eine -O- oder -NH-, -S- oder -CO-Gruppe ersetzt sein kann. Bevorzugt sind zwei-atomige Brücken
   Ganz besonders bevorzugt ist eine -CH2-CH2-, -CH2-NH- oder -NH-CO-Gruppe. Wird eine Peptid-CO-NH-Gruppe beispielsweise durch eine -NH-CO-Gruppe ersetzt, erhält man formal aus zwei Aminosäuren des natürlichen HCV-Epitops ein Diamin und eine Dicarbonsäure. Wird eine -CO-NH-Gruppe beispielsweise durch eine -CH2-CH2-Gruppe ersetzt, erhält man formal innerhalb der Sequenz der erfindungsgemaßen Bindungssubstanz ein Aminovaleriansäurederivat.
b)
   Verlängerung oder Verkürzung der Seitenketten einer oder mehrerer Aminosäuren des natürlichen HCV-Epitops, so daß keine natürlichen Aminosäurereste entstehen. Bevorzugt ist dabei der Einbau einer zusätzlich -CH2-, -S-, -O-, -NH-, -SO2-, -CO-Gruppe oder das Weglassen von β-Seitenketten aller Aminosäuren des Epitops durchgeführt. Wird nur eine Seitenkette verändert, soll dabei keine natürliche Aminosäure entstehen.
c)
   Ersatz einer natürlichen Aminosäure des natürlichen HCV-Epitops durch eine oder mehrere nichtbiogene L- oder D-Aminosäuren des Typs
   HR 'N-Y-COOH
   wobei
   R' Wasserstoff und
   Y eine (CH2)n Gruppe darstellt, mit n=2-8 oder
   eine -CH-(CH2)m-CR¹R²R³ -Gruppe darstellt mit m=0-3, wobei
   R1, R² und R³, die gleich oder verschieden sein können, Wasserstoff, einen verzweigten oder unverzweigten C1-C4-Alkylrest darstellen oder einen Phenyl-, Naphtyl- oder einen O, S oder N enthaltenden 5-6 gliedrigen Heteroarylrest darstellen, der mit Merthyl, Halogen, NH2. OH oder Carboxy substituiert sein kann oder wobei
   Y eine CHR-Gruppe darstellt, wobei
   R eine Seitenkette einer natürlichen Aminosäure darstellt, in der eine -CH2-Gruppe durch - S-, NH-, -CH=-, -SO2-, -CO- oder -O- ersetzt ist, oder in der ein oder mehrere H-Atome durch CH₃, NH₂, Carboxyl, SH, Halogen oder Hydroxy ersetzt sind.
      wobei in allen Fallen Y eine in keiner natürlichen Aminosäure vorkommende Gruppierung bildet oder
      wobei R' Methyl, Ethyl oder Phenyl und
   Y eine CHR Gruppe darstellt in der R eine Seitenkette einer natürlichen Aminosäure ist.

Unter Halogen wird dabei F, Cl, Br oder Jod, insbesondere Cl verstanden. Unter Heteroarylresten sind besonders Pyridin, Pyrrol, Furan und Thiophen bevorzugt.

Die erfindungsgemaße Bindungssubstanz bindet solche Antikörper, die gegen ein Antigen mit einem entsprechenden HCV-Epitop aus einer natürlichen Aminosäuresequenz gerichtet sind. Beispiele sind die Epitope des HCV Virus:

Unter Epitop mit einer natürlichen Aminosäuresequenz wird die Mindestaminosäuresequenz bestehend aus natürlichen Aminosäuren eines natürlichen Antigens, zum Beispiel eines infektiösen Organismus, verstanden, die für die Bindung eines durch das Antigen erzeugten Antikörpers notwendig ist. Dies sind Aminosäuresequenzen aus mindestens 6 Aminosäuren, bevorzugt mindestens 10 Aminosauren. Selbstverständlich können sich an die Epitopaminosäuresequenz am C- oder N-terminalen Ende weitere Aminosäuren anschließen. Diese tragen aber nicht mehr essentiell zur Bindung des Antikörpers bei. Die erfindungsgemäße immunologische Bindunussubstanz soll mindestens dieser Aminosäuresequenz des natürlichen HCV-Epitops entsprechen, wobei sie allerdings innerhalb dieser Aminosäuresequenz mindestens an einer Stelle in der erfindungsgemäßen Weise verändert ist.

Bevorzugt wird eine -CO-NH-Gruppe einer Peptidbindung erfindungsgemäß ersetzt, ganz besonders bevorzugt an einer Stelle der Sequenz an der die zugehörige Amidbindung durch eine Protease besonders spaltungsgefährdet ist. Dabei muß nicht an jeder möglichen Proteasespaltstelle eine -CO-NH-Gruppe durch eine hydrolysestabile Atomgruppe modifiziert werden. Dem Fachmann sind die Konzentrationen und Arten relevanter Proteasen in verschiedenen Körperflüssigkeiten bekannt. Häufig vorkommende Proteasen sind beispielsweise Trypsin, Chymotrypsin, Collagenase, Elastase, Thrombin, Plasmin oder Kallikrein (z.B. N. Katunuma et al. Japan. Sci. Soc. Press. Tokyo / Springer Verlag Berlin, Seite 37-44 (1983). Ebenso sind ihm die jeweiligen Aminosäuresequenzen der Spaltstellen verschiedener Proteasen bekannt. Daneben gibt es auch unspezifische Proteasen. Besonders spaltungsgefährdet sind beispielsweise Proteasespaltstellen an den Enden einer Aminosäuresequenz. Hier ist die Aminosauresequenz besonders gefährdet durch den Abbau durch Exopeptidasen. Weiter sind Glycin enthaltende Peptidsequenzen besonders spaltungsgefährdet, da die fehlende Seitengruppe des Glycins einen hydrolytischen Angriff einer unspezifischen Protease besonders begünstigt. Durch die erfindungsgemäße Modifikation einer durch Peptidasen gefährdeten Amidbindung, insbesondere einer Glycin-Peptid-Bindung allein, kann schon eine wesentlich verbesserte Stabilität der erfindungsgemäßen Bindungssubstanzen gegenuber den natürlichen HCV-Epitopen bezüglich Abbau durch Proteasen erreicht werden.

Die Proteasestabilitat einer erfindungsgemaßen Bindungssubstanz kann vorteilhaft beispielsweise so ermittelt werden, daß sie Pankreatin, einem Gemisch häufig vorkommender Proteasen (u.a. Carboxypeptidase A und B, Trypsin, Pepsin, Chymotrypsin, Elastase) oder Pronase (u.a. Aminopeptidase, Carboxypeptidase, alkalische und neutrale Proteinase), ausgesetzt wird. Der verminderte enzymatische Abbau der Bindungssubstanz kann durch HPLC-Analyse des zeitlichen Auftretens von Peptidbruchstücken bestimmt werden.

Ganz besonders bevorzugt wird eine -CO-NH-Gruppe durch -CH2-CH2-, CH2-NH- oder NH-CO-Gruppen ersetzt.

Eine zusatzliche Blockierung der Sequenz-Termini stabilisiert die Bindungssubstanz zusätzlich gegenüber Exopeptidasen.

Die immunologische Bindungssubstanz ist entweder an ein detektierbares Markierungsmolekül, an eine Festphase oder an ein spezifisches Bindungsmolekül, das an eine Festphase spezifisch binden kann, gekoppelt. Diese Kopplung erfolgt bevorzugt kovalent, entweder direkt über das C-oder N-terminale Ende der Bindungssubstanz oder über einen Spacer.

Eine detektierbare Markierung kann direkt oder indirekt ein messbares Signal liefern, zum Beispiel durch Radioaktivitat. Chemilumineszenz, Phosphoreszenz, Fluoreszenz oder Elektrochemilumineszenz oder durch sichtbare Farbe. Ein indirekt messbares Signal liegt zum Beispiel bei Enzymmarkierung vor. Hier wird durch Zugabe eines Enzymsubstrates eine Farbe gebildet. Beispiele für Enzymmarkierungen sind: Markierungen mit β-Galaktosidase, alkalische Phosphatase oder Peroxidase.

Die Bindung der Bindungssubstanz an eine Festphase erfolgt nach dem Fachmann geläufigen Methoden. Als Festphasen dienen beispielsweise Kugein. Kunststoffröhrchen, Mikrotiterplatten oder Testträger.

Die immunologische Bindungssubstanz kann auch an ein spezifisches Bindemolekül gekoppelt sein, über das es an eine Festphase binden kann. Ein bevorzugtes Beispiel ist Biotin, das bevorzugt am N-terminalen Ende der Bindungssubstanz gebunden ist und spezifisch an eine mit Streptavidin beschichtete Oberfläche binden kann.

Die erfindungsgemäße Bindungssubstanz kann vorteilhaft in Immunoassays eingesetzt werden. Besonders vorteilhaft ist ihr Einsatz in Immunoassays nach dem Sandwichprinzip zum Nachweis eines Antikörpers, der gegen ein HCV-Epitop mit einer entsprechenden, natürlichen Aminosäuresequenz gerichtet ist, durch Inkubation der Antikörperprobe mit zwei Bindungspartnern, die mit dein Antikörper spezifisch binden, und Bestimmung der Bildung des Antikörper-Bindungspartner-Komplexes in einer geeigneten Weise, dadurch gekennzeichnet, daß mindestens einer der Bindungspartner eine erfindungsgemäße Bindungssubstanz darstellt.

Bevorzugt wird ein solcher Immunoassay als heterogener Immunoassay durchgeführt. Dabei ist einer der Bindungspartner entweder direkt oder über einen Spacer an eine Festphase gebunden oder ist an eine spezifische Bindungsstelle. zum Beispiel Biotin, gebunden, die während des Immunoassays an die Festphase binden kann. Für diesen Bindungspartner wird eine erfindungsgemäße Bindungssubstanz verwendet.

Der zweite Bindungspartner ist markiert. Für diesen Bindungspartner kann entweder eine erfindungsgemäße, mit einem Markierungsmolekül versehene Bindungssubstanz oder ein gegen den nachzuweisenden Antikörper gerichteter, markierter Anti-Antikörper benutzt werden. Bevorzugte Markierungen sind Enzymmarkierungen oder Direktmarkierungen mit beispielsweise Metallsolen.

Zur Durchführung des Immunoassays wird eine Probe des nachzuweisenden Antikörpers mit dem ersten Bindungspartner, dem markierten Bindungspartner und der Festphase gleichzeitig oder sukzessive inkubiert, wobei sich ein festphasengebundener Komplex aus erstem Bindungspartner, Antikörper und markiertem Bindungspartner bildet. Bevorzugt werden dann nichtgebundene. markierte Bindungspartner in der flüssigen Phase von an der Festphase gebundenen, markierten Bindungspartnern getrennt und die Markierung in einer der beiden Phasen als Maß für die Anwesenheit oder Konzentration des nachzuweisenden Antikörpers gemessen. Bei Enzymmarkierungen beispielsweise wird zur Messung die Enzymsubstratlösung dazugegeben. Die Messung kann beispielsweise visuell oder photometrisch erfolgen.

Ein weiterer Gegenstand der Erfindung ist ein Immunogen bzw. ein Vakzin enthaltend eine erfindungsgemäße Bindungssubstanz, die an eine Aminosäuresequenz, die ein T-Zell-Epitop enthält, oder an ein Carrier-Molekül gekoppelt ist, wobei die Bindungssubstanz mit einem Antikörper, der gegen ein HCV-Epitop mit einer natürlichen Aminosäuresequenz gerichtet ist, spezifisch bindet und zusammen mit dem Carrier-Molekül fähig ist, solche Antikörper als Immunantwort zu erzeugen.

Das Vakzin dient zur vorbeugenden Behandlung von Infektionen, die durch Antigene hervorgerufen werden, die ein HCV-Epitop der jeweiligen natürlichen Aminosäuresequenz enthalten. Zum Einsatz als Immunogen muß die erfindungsgemäße Bindungssubstanz am C- oder N-terminalen Ende entweder an ein geeignetes hochmolekulares Trägerprotein, wie zum Beispiel Keyhole limpet hemocyanine, Hemocyanine, Rinderserumalbumin oder Edestin gebunden werden. Die Kopplung kann zum Beispiel am N-terminalen Ende der Aminosäuresequenz über Maleinimidohexansaure-N-Hydroxysuccinimidester erfolgen. Da im allgemeinen Vakzine (Immunogene) auch T- oder B-Zell-Epitope sind, die peptidischer Natur sein können, kann die erfindungsgemaße Bindungssubstanz auch an eine Aminosauresequenz gekoppelt werden, die T-Zell-Epitope enthält.

Das Vakzin liegt in einer pharmakologisch effektiven Dosis und einer pharmazeutisch akzeptablen Formulierung vor.

Obwohl in einer Immunantwort Antikörper gegen ein künstliches Epitop mit einer vom natürlichen Epitop unterschiedlichen Aminosäurestruktur erzeugt werden, binden diese Antikörper auch an das naturliche Epitop und konnen so zur Immunabwehr gegen Antigene, die dieses Epitop enthalten, verwendet werden. Aufgrund der Veranderung in der Sequenz zeigen sie aber ein verändertes Verhalten in Bezug zu ihrem Metabolismus im tierischen Organismus, z.B. eine veränderte Proteasestabilität.

Mit dem erfindungsgemäßen Immunogen ist es auch möglich, durch geläufige Verfahren der Immunisierung Antikörper zu erhalten mit denen Antigene, die die Aminosäuresequenz der natürlichen HCV-Epitope enthalten, in einem immunologischen Bestimmungsverfahren nachgewiesen werden konnen.

Ein weiterer Gegenstand der Erfindung ist deshalb ein Verfahren zur Herstellung von Antikörpern, die gegen ein HCV-Epitop einer natürlichen Aminosäuresequenz gerichtet sind, das dadurch gekennzeichnet ist, daß ein Säugetier mit einer entsprechenden erfindungsgemäßen Bindungssubstanz, das mit einem Molekül verbunden ist, das ein T-Zell-Epitop enthält. z.B. ein Carrierproteinmolekül, immunisiert wird und die erzeugten Antikörper nach bekannten Verfahren, z.B. aus dem Serum oder der Milz gewonnen werden.

Die erfindungsgemäßen Bindungssubstanzen zeigen zum einen ein verändertes metabolisches Verhalten im Organismus, insbesondere eine veranderte Proteasestabilität. Trotz ihrer Modifizierung binden sie selektiv in einem Immunoassay gegen Antikörper, die gegen das natürliche HCV-Epitop gerichtet sind. Überraschenderweise wurde zusätzlich festgestellt, daß die erfindungsgemaßen Bindungssubstanzen, insbesondere solche mit einem Ersatz einer CONH-Gruppe ("backbone"-Modifizierung), bei der Verlaufskontrolle frisch Infizierter zum Teil früher die Serumkonversion anzeigen. Außerdem wird durch die erfindungsgemäßen Bindungssubstanzen, insbesondere durch solche mit Seitenkettenmodifizierung, ein spezifischerer Nachweis von Null-Seren moglich, wahrend die Empfindlichkeit bei positiven Seren vergleichbar mit den natürlichen HCV-Epitopen ist.

Die Herstellung der erfindungsgemaßen Bindungssubstanz ist nach den dem Fachmann geläufigen Methoden zur Peptidsynthese möglich (z.B. Merrifield. JACS 85 (1964), 2146). Ein weiterer Gegenstand der Erfindung ist deshalb ein Verfahren zur Herstellung der erfindungsgemäßen Bindungssubstanzen, welches darin besteht, daß die das C-terminale Ende bildende Aminosäure an einen Träger gebunden wird, vom C-terminalen Ende die gewünschte Aminosäuresequenz schrittweise aufgebaut wird, diese anschließend vom Träger abgespalten wird und vor oder nach der Abspaitung vom Träger an ein Markierungsmolekül, an ein spezifisches Festphasen-Bindemolekül, an eine Festphase oder an ein Carriermolekül gekoppelt wird, dadurch gekennzeichnet, daß für die Peptidsynthese an der Stelle mindestens einer Pepridbindung der natürlichen HCV-Epitopsequenz statt der nacheinander diese Peptidbindung aufbauenden ersten Aminosäure NH₂-CHR¹-COOH und zweiten Aminosäure NH₂-CHR²-COOH eine nichtnatürliche Aminosäure NH2-CHR¹-X-CHR²-COOH verwendet wird, in der R¹ und R², die gleich oder verschieden sein können. Seitenketten von naturlichen Aminosäuren bilden und X eine proteasestabile Atomgruppierung, insbesondere eine zwei- bis dreiatomige Kette wie eine -NH-CO-, -CH₂-CH₂-, -CH=CH-, - NH-CH₂-, CH₂-NH, CH₂-O-, O-CH₂-, CH₂-S-, -S-CH₂- oder -CH₂-CH₂-CH₂-Gruppe darstellt.

Ist die Modifikation die Veränderung einer Aminosaureseitenkette oder der Ersatz einer natürlichen Aminosaure des naturlichen HCV-Epitops durch eine künstliche, nicht natürliche Aminosaure so ist das erfindungsgemaße Herstellverfahren dadurch gekennzeichnet, daß statt der zum HCV-Epitop gehörenden natürlichen Aminosäure eine entsprechend modifizierte Aminosäure benutzt wird, so daß kein natürlicher Aminosäurerest entsteht. Diese künstlichen Aminosäuren sind teilweise käuflich erhältlich oder können nach dem Fachmann bekannten Methoden synthetisiert werden, (z.B. L. Gazerro et al. Solid phase synthesis. Seite 403, 1990; G. Jung, Peptides 1988, Walter de Gruyter, Berlin, New York 1989, Seite 646 - 648; A. Jäger, Peptides 1992, Escom Science Publisher B.V. 1993, Seite 47-49, R. Varrel, Peptides 1990, Escom Science Publishers B.V. 1991, Seite 642-664, Seite 393-394, Seite 385-386, Seite 370-371).

Im einzelnen wird zur Synthese der Aminosäuresequenz eine Aminosäure des C-terminalen Ende über ihre Carboxygruppe an ein unlösliches, leicht filtrierbares Polymer geknüpft, und dann vom C-terminalen Ende her die Peptidkette schrittweise aufgebaut. Zu diesem Zweck wird eine N-geschützte Aminosäure mit einer reaktiven Gruppierung des Polymeres zur Reaktion gebracht. Von der am Trägermaterial kovalent verankerten Aminosäure wird die N-α-Schurzgruppe entfernt und das resultierende Aminoacylpolymer mit der nächsten N-geschützten Aminosäure umgesetzt, Von dem am Träger kovalent gebundenen Dipeptid wird die N-α-Schutzgruppe entfernt und das resultierende Aminoacylpolymer mit der nächsten N-geschützten Aminosäure umgesetzt. Alle überschüssigen Reagenzien und Beiprodukte werden durch einfaches Filtrieren entfernt. Ist die gewünschte modifizierte Peptidsequenz auf diese Weise hergestellt, wird die kovalente Bindung zwischen der C-terminalen Aminosaure und der Ankergruppierung des polymeren Trägers gespalten. Der unlösliche Träger wird durch einfache Filtration von dem in Lösung befindlichen Peptid entfernt. Das Peptid wird mittels chromatographischer Methoden gereinigt. Die Kopplung des Peptids an eine Festphase, an ein Carriermolekül, an eine spezifische Festphasen-Bindungsstelle oder an eine Markierung erfolgt nach bekannten Methoden, bevorzugt am N-terminalen Ende der Peptidsequenz. Eine Biotinylierung kann beispielsweise nach PNAS USA 80, 1983, 4045 erfolgen. Ein bevorzugtes Biotinylierungsmittel ist Biotinylaminocapronsaure-N-Hydroxysuccinimidester. Diese Gruppen können auch schon am N-Terminus der modifizierten Aminosauresequenz am Ende der Festphasensynthese eingeführt werden.

### Beispiel 1:

Durchführung eines Immunoassays mit erfindungsgemäßen biotinylierten Bindungssubstanzen gegen Antikörper gerichtet gegen das core-Epitop des HCV-Virus.

In den eingesetzten Bindungssubstanzen wurde gegenüber dem natürlichen Epitop "core 2m" eine oder mehrere CO-NH-Peptidbindungsgruppen erfindungsgemäß ersetzt (core2m I - core2m VI).

In streptavidinbeschichteten ES 22 Tubes der Firma Boehringer Mannheim werden jeweils 20 µl Serum vorgelegt (Negativserum, Positivserum, positive Seren A-C). Dann wird 1000 µl Peptidlösung (core 2m bzw. core 2m I-VII, Konzentration 100ng / ml in Kaliumphosphatpuffer 40 mmol / 1, pH 7.0) zupipettiert und 60 min im ES 22-Gerät der Firma Boehringer Mannheim stehengelassen. Nach 60 min Inkubation wird die Peptid / Serumlösung herauspipettiert und die Tubes mit Testwaschlösung Kaliumphosphatpuffer 40 mmol /1, pH 7,0 gewaschen. Dann wird 1000 µl Peroxidasekonjugatlösung (Peroxidase konjugiert an einen Anti FcY-Antikoper. 0.5 U/ml) zupipettiert und wieder 60 min inkubiert. Die Konjugatlösung wird abpipettiert und das Tube mit Waschlösung gewaschen. Dann wird 1000 µl ABTS-Lösung (1,9 mmol /l) zupipettiert und wieder 60 min inkubiert. Nach 60 min Inkubation wird die Lösung aus dem Tube in ein Photometer pipettiert und bei 405 nm gemessen. Tabelle 1 zeigt die relativen Reaktivitäten der künstlichen Epitope core2m I-VI im Vergleich zum naturlichen Epitop (core 2m). Die Reaktivitaten ergeben sich aus dem Messergebnis des Immunoassay in Bezug zu einer Standardkurve. Trotz der Modifikation binden die künstlichen Epitope spezifisch und selektiv (zum Beispiel core 2m I).

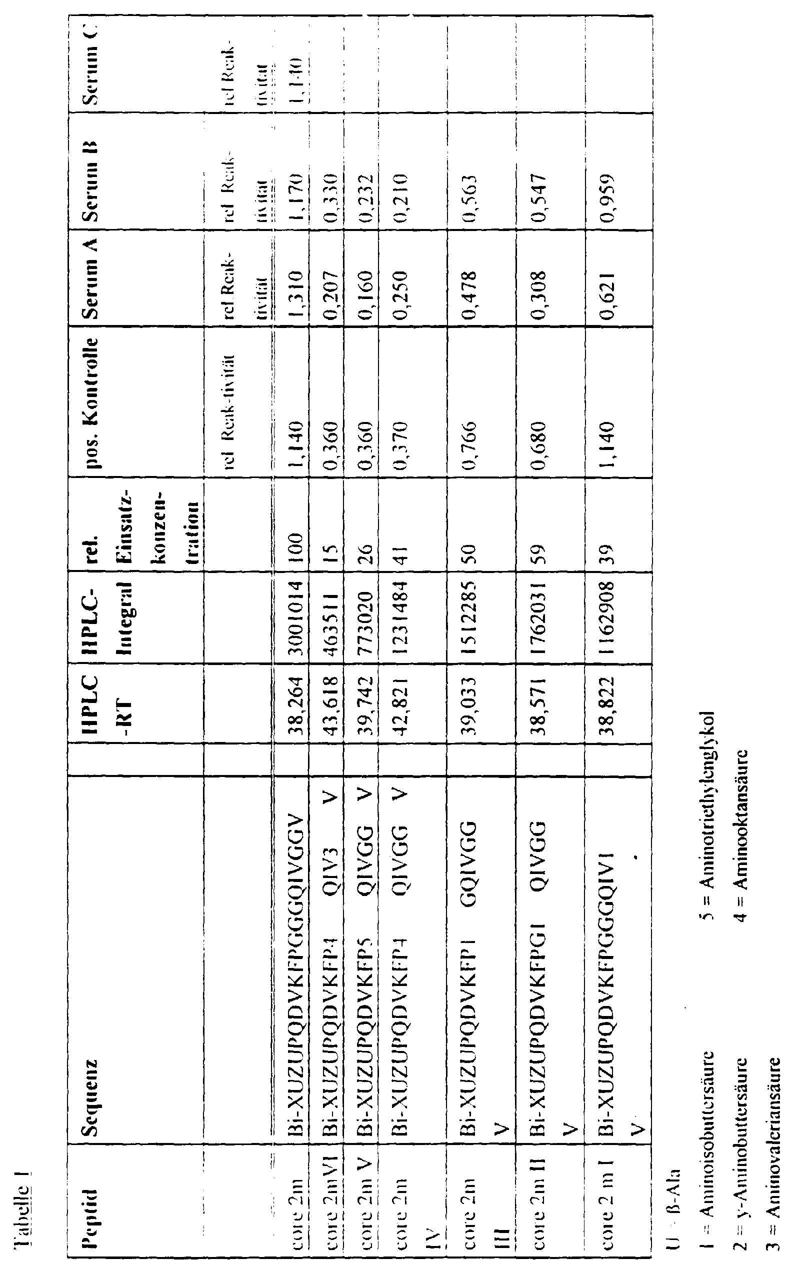

### Relative Reaktivität

Die relative Einsatzkonzentration der Peptidantigene I-VI ergab sich aus deren HPLC-Intetral.
Die Reaktivitat der Antigene I-VI wurde relativ zum Referenzantigen Core2m gleicher Konzentration ermittelt.

### Beispiel 2:

Versuchsdurchführung für core2m-Verdau mit Pankreatin

Es wurden zuerst core2m-Lösungen mit einem ungefähren c=1-3 mg / ml in destilliertem Wasser hergestellt. Diese Lösungen wurden in die anal. HPLC eingespritzt (Injektionsvolumen = 40 µl).

Diese Lösungen wurden nun so verdünnt, daß bei erneutem Einspritzen in die HPLC die Fläche 40 000 betragen mußte.
Z.B. Fläche der core2m IV-Lösung ergab 600 000 auf der anal. HPLC bei 40 µl Injektionsvolumen.
Nun werden zwei Teile dieser Lösung mit einem Teil destilliertem Wasser verdünnt.

Jeweils 200 µl dieser eingestellten core2m-Lösungen wurden mit 50 µl Pankreatin-Lösung (c = 1 mg / ml) versetzt.

Von diesen Losungen wude nach 55 min. und nach 125 min. eine Probe in die anal. HPLC eingespritzt. Als Referenz wurde von jeder core2m-Lösung eine 200 µl-Probe mit 50µl destilliertem Wasser versetzt und in die anal. HPLC eingespritzt.

Über die Flächen des Peptidpeaks der anal. HPLC kann man den prozentualen Abbau des jeweiligen Peptids bestimmen.

Tabelle 2 gibt den prozentualen Anteil in % der Resistenz der einzelnen Epitopsequenzen gegenüber Proteaseabbau nach 55 min. und 125 min. an. Während das natürliche Epitop "core2m" vollständig abgebaut wird (0% Resistenz) sind die künstlichen Epitope core2m I-VI wesentlich proteasestabiler.

**Tabelle 2**

| HCV Core2m-Belastung mit Pankreatin | | | | | | | |
|---|---|---|---|---|---|---|---|
| Core2m-Lösungen mit HPLC-Areas auf 400 000 in 40 µl Injektionsgröße eingestellt Pankreatin-Lösung c=1mg/ml in H2O 200 l der eingestellten core2m-Lösung mit 50µl Pankreatin-Lösung reagieren lassen | | | | | | | |

| **Probe** | **Sequenz** | **Area vorher** | **%** | **Area nach 55 min.** | **%** | **Area nach 125 min.** | % |
|---|---|---|---|---|---|---|---|
| core2m | Bi-XUZUPQDVKFPGGGQIVGGV | 3163895 | 100 | 0 | 0 | 0 | 0 |
| core2m I | Bi-XUZUPQDVKFPGGGQIVI V | 3240090 | 100 | 3252704 | 100 | 2460332 | 76 |
| core2m II | Bi-XUZUPQDVKFPGI QIVGGV | 3114243 | 100 | 561742 | 18 | 110690 | 4 |
| core2m III | Bi-XUZUPQDVKFPI GQIVGGV | 3265196 | 100 | 657676 | 20 | 135624 | 4 |
| core2m IV | Bi-XUZUPQDVKFP4 QIVGGV | 3415399 | 100 | 3057359 | 90 | 2715127 | 80 |
| core2m V | Bi-XUZUPQDVKFP2 QIVGGV | 2992631 | 100 | 2142962 | 72 | 765326 | 26 |
| core2m VI | Bi-XUZUPQDVKFP4 QIVI V | 366701 | 100 | 2551154 | 69 | 1453596 | 39 |

### Beispiel 3:

### Synthese des serumstabilen HCV-Antigens core2m I

Das Antigen wurde mittels Fmoc-( Fluorenylmethoxycarbonyl-)-Festphasenpeptidsynthese mit einem SMPS 350 Peptidsynthesizer der Firma Zinsser Analytics an 15 mg 4-(2',4'-Dimethoxyphenyl-Fmoc-aminomethyl)-phenoxy-Harz SA-5030 der Firma Advanced Chemtech mit einer Beladung von 0,52 mmol/g hergestellt. Von folgenden N-Fmoc-Aminosäure Derivaten wurden nacheinander zweimal je 90 µmol zusammen mit je 90 µmol 1-Hydroxybenzotriazol in 270 µl Dirnethylformamid und 105 µmol einer Dimethylformamidlösung von 90 µmol N,N-Diisopropylcarbodiimid an das aufzubauende festphasengebundene Peptid angekoppelt: Valin, δ-Aminovaleriansäure, Valin, Glycin, Glycin, Glycin, Prolin, Phenylalanin, Lysin (tert. Butyloxycarbonyl), Asparaginsäure (tert. Butylester), Glutamin (Trityl), Prolin, β-Alanin, ε-Aminocarpronsäure, β-Alanin, tert. Butyloxycarbonyl-Lysin, Dimethoxytritylbiotin. Die Kupplungszeiten betragen 40 und 50 Minuten. Die Abspaltungszeit der Fmoc-Schurzgruppe erfolgt nach jeder Doppelkupplung mit 600 µl einer 50%igen Lösung von Piperidin in Dimethylformamid. Die Abspaltzeit beträgt 20 min. Die Waschschritte werden mit jeweils 700 µl Dimethylformamid achtmal nach jedem der Reaktionsschrirte durchszeführt. Die Freisetzung des Peptids erfolgt durch Behandlung des vom Lösungsmittel filtrierten Harzes mit je 750 µl einer Mischung as 90% Triflouressiusaure, 3% Thioanisol, 3% Ethandithiol und 3% Thiokresol innerhalb von 20 Minuten und abschließend 140 Minuten. Das Produkt wird durch Zugabe von 15 ml kaltem Diisopropylether zum vereinigten Filtrat ausgefällt und durch Filtration isoliert. Der Rückstand wird in 3 ml 50%iger Essigsäure gelöst und lyopohilisiert. Der Lyophilisationsvorgang wird zweimal wiederholt. Es werden 17 mg Rohmaterial einer Reinheit von 94% laut reverse Phase HPLC erhalten (LSIMS: M-H⁺, Matrix: mNBA, Beschleunigungsspannung: 6 kV)

### Beispiel 4 :

Synthese eines tetrameren HCV-Vakzinbestandteils mit erhöhter Resistenz gegen Proteasen:

Das Vakzin besteht aus einem B-Zell Epitop des HCV-Antigens (HCV core 18-33) gekoppelt an ein T-Zell-Epitop aus einem Ebstein-Barr-Virus (EBV LMP 43-53), wobei in beiden Epitopen eine Glycin-Glycin-Peptidbindung des natürlichen Epitops durch eine - CH2-CH2-Gruppe ersetzt ist (entspricht dein Einbau einer δ-Aminovalerian-Säure)

Die synthetische Vaccine wurde mittels Fmoc-(Fluorenylmethoxycarbonyl-)-Festphasenpeptidsynthese mit einem SMPS 350 Peptidsynthesizer der Firma Zinsser Analytics an 15 mg 4-(2',4'-Dimerhoxyphenyl-Fmoc-aminomethyl)-phenoxy-Harz SA-5030 der Firma Advanced Chemtech mit einer Beladung von 0,22 mmol/g hergestellt. Von folgenden N-Fmoc-Aminosäure Derivaten wurden nacheinander zweimal je 90 µmol zusammen mit je 90 µmol 1-Hydroxybenzotriazol in 270 µl Dimethylformamid und 105 µmol einer Dimethylformamidlosung von 90 µmol N,N-Diisopropylcarbodiimid an das aufzubauende festphasengebundene Peptid angekoppelt: Nε-Fmoc-Lysin, Nε-Fmoc-Lysin, β-Alanin, β-Alanin, Leucin, Leucin, Alanin, δ-Aminovaleriansaure, Threonin(-tert.Buthylester), Tryptophan, Asparaginsaure (-tert. Butylester), Serin (tert. Butylester), Methionin, Valin, Valin, δ-Aminovaleriansäure, Valin, Isoleucin, Glutamin (Trityl), Glycin, δ-Aminovaleriansäure, Prolin, Phenylalanin, Lysin (tert. Butyloxycarbonyl), Valin, Asparaginsäure (tert. Butylester), Glutamin (Trytil), Prolin, Essigsäure. Die Kupplungszeiten betragen 40 und 50 min. Die Abspalrungszeit der Fmoc-Schutzgruppe erfolgt nach jeder Doppelkupplung mit 600 µl einer 50%igen Losung von Piperidin in Dimethylformamid. Die Abspaltzeit betraut 20 Minuten. Die Waschschritte werden mit jeweils 700 µl Dimethylformamid achtmal nach jedem der Reaktionsschritte durchgeführt. Die Freisetzung des Peptids erfolgt durch Behandlung des vom Lösungsmittel filtrierten Harzes mit je 750 µl einer Mischunu aus 90% Triifluoressigsaure, 3% Thioanisol, 3% Ethandithiol und 3% Thiokresol innerhalb von 20 Minuten und anschließend 140 Minuten. Das Produkt wird durch Zugabe von 15 ml kaltem Diisopropyiether zu vereinigtem Filtrat ausgefällt und durch Filtration isoliert. Der Rückstand wird in 3 ml 50%iger Essigsäure gelöst und lyophilisiert. Der Lyophilisationsvorgang wir zweimal wiederholt. Es werden 13 mg Rohmaterial einer Reinheit von 42% laut reverse phase HPLC erhalten, wovon 4 mg mittels praparativer reverse phase HPLC gereinigt werden. Ausbeute: 0,7mg (LSIMS: M-H⁺; Matrix: mNBA, Beschleunigungsspannung: 6 kV).

### Beispiel 5

Reaktivität verschiedener seitenkettenmodifizierter erfindungsgemäßer Bindungssubstanzen ("Mimetope") des HCV-Epitops "core 1" ["12 D1", in Tabelle 3 und Figur 1 und 2] und von peptidbindungsmodifizierten Epitopen des HCV-Epitops "core2" ["1B2" in Tabelle 4 und Figur 3].

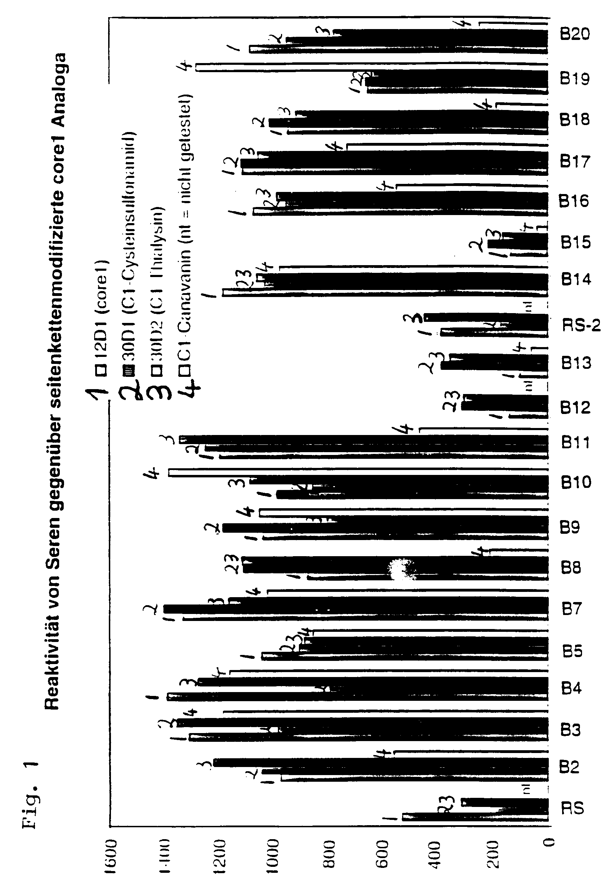

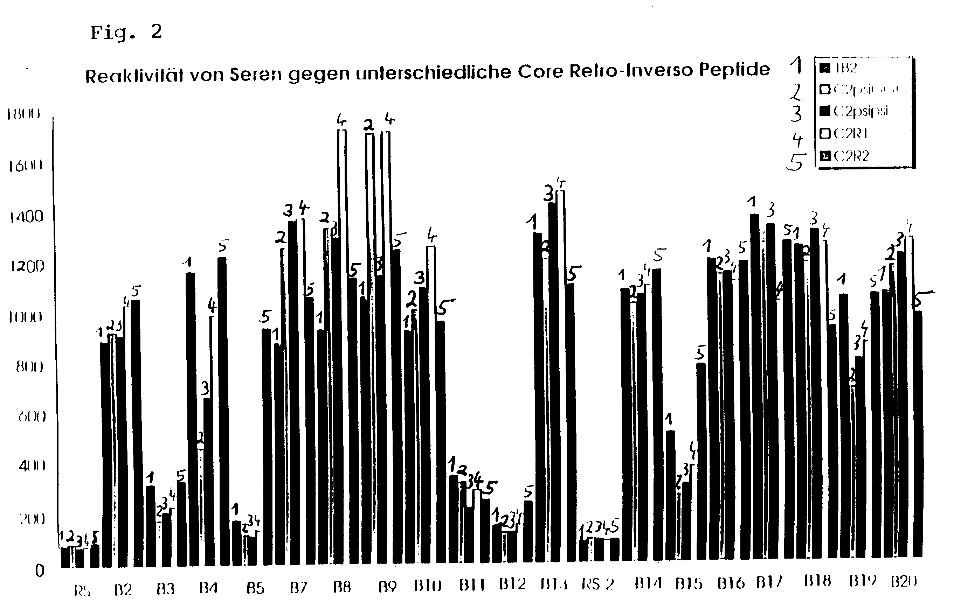

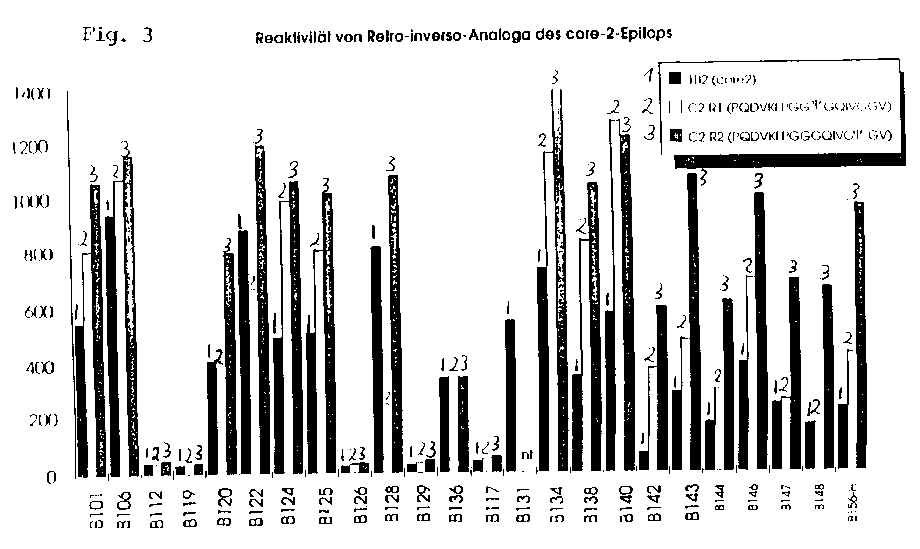

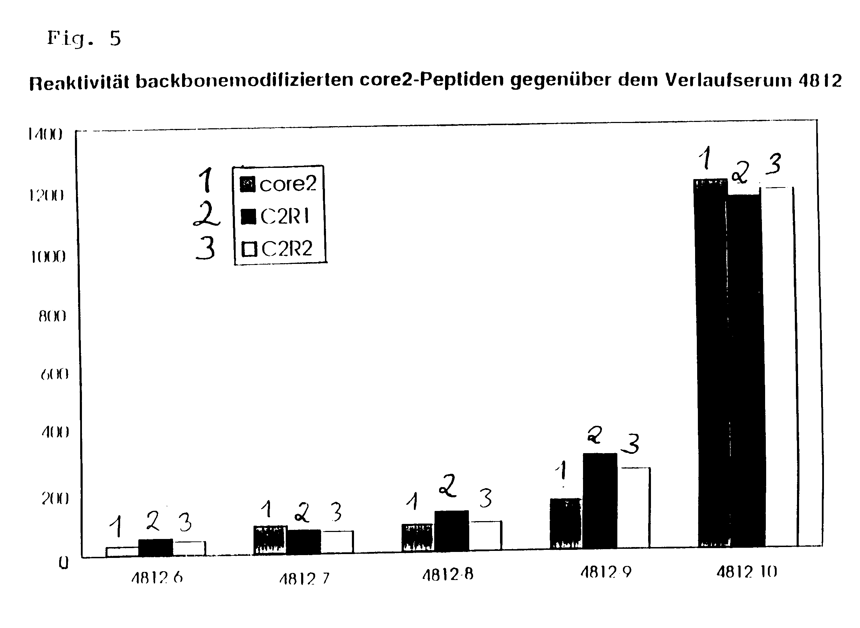

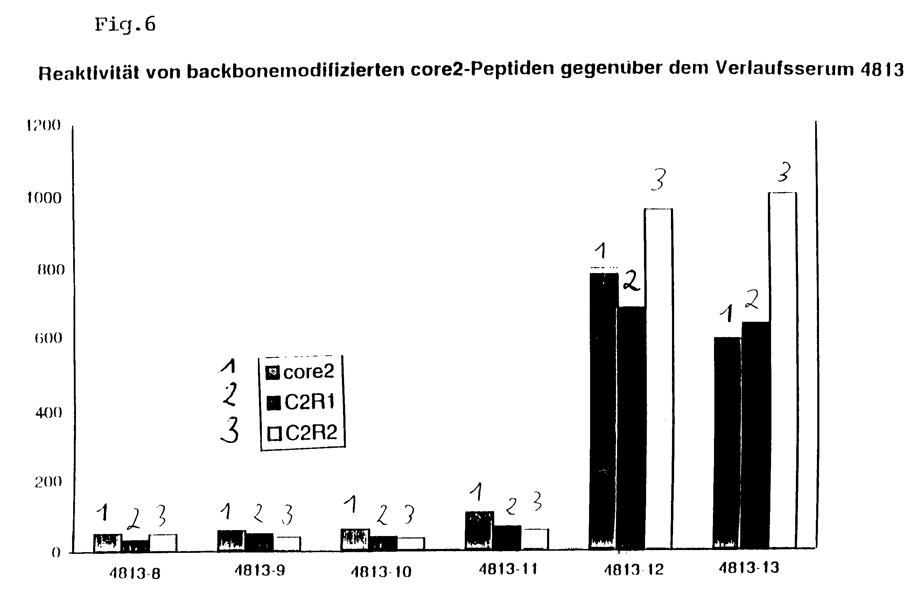

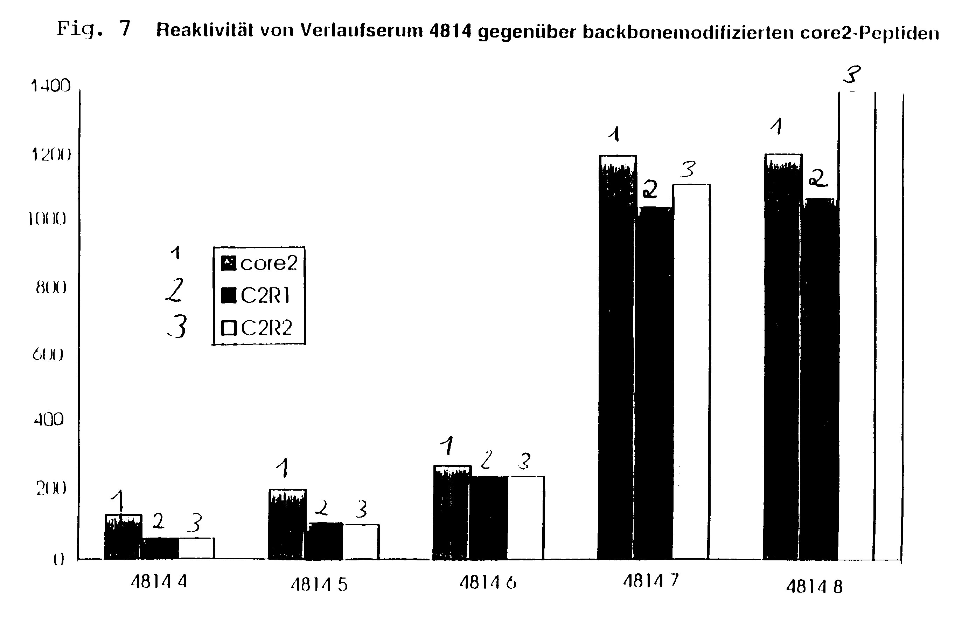

Die Reaktivitaten der Mimetope sind für verschiedene Seren B3 bis B20 im Vergleich zum Standard Antigen core1 (12 D1) bzw. core2 (1B2) dargestellt (Figuren 1 bis 7). Die Seren RS und RS2 (Figuren 1 und 2) bzw. B112, B119, B126, B129 und B117 (Figur 3) sind Negativseren. In Figur 1 ist zu erkennen, daß Negativseren mit seitenkettenmodifizierten Mimetopen spezifischer als solche erkannt werden.

Die Mimetope zeigen vergleichbare Reaktivitaten zu natürlichen Epitopen, teilweise sogar höhere Reaktivitaten.

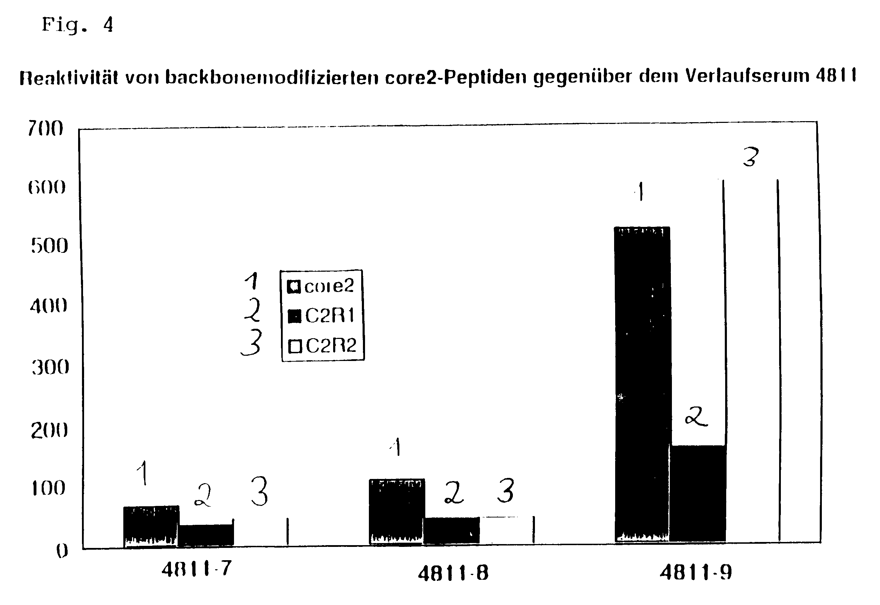

In Figuren 4 bis 7 ist zu erkennen, daß bei der Verlaufskontrolle von Seren kürzlich infizierter Personen vergleichbar früh oder sogar früher die Serumkonversion mit Mimetopen erkennbar ist.

Die Reaktivitaten wurden analog Beispiel 1 bestimmt.

**Tab. 3**

| HCV-Mimetope von Core 1 (12D1 KKNKRNTNRR) | | | |
|---|---|---|---|
| Mimetop | Struktur | Beschreibung | Bewertung |
| 30D2 | TlyTlyAsnTlyArgAsnThrAsnArgArg | Seitenkettenmimetop K→Thialysin(Tly) | s. Fig 1 |
| 30D1 | LysLysCsaLysArgCsaThrCsaArgArg | Seitenkettenmimetop N→Cysteinsulfonamid(Csa) | s. Fig 1 |
| C1-Canavanin | LysLysAsnLysCanAsnThrAsnCanCan | Seitenkettenmimetop R→Canavanin(Can) | s. Fig 1 |

**Tab. 4**

| HCV-Mimetope von Core 2 (1B2: PQDVKFPGGGQIVGGV) | | | |
|---|---|---|---|
| Mimetop | Struktur | Beschreibung | Bewertung |
| C2R1 | PQDVKPGGΨGQIVGGV | Peptidbindungsmimetop G-G→G(ψNH-CO)G | s. Fig 2,3,4,5,6 und 7 |
| C2R2 | PQDVKFPGGGQIVGΨGV | Peptidbindungsmimetop G-G→G(ψNH-CO)G | s. Fig 2,3,4,5,6 und 7 |
| C2psiGGG | PQDVKFPGΨGΨGQIVGGV | Peptidbindungsmimetop G-G→G(ψNH-CO)G | s. Fig 3 |
| C2psipsi | PQDVKFPGGΨGQIVGΨGV | Peptidbindungsmimetop G-G→G(ψNH-CO)G | s. Fig. 3 |

### Vergleichsbeispiel 6

Durchführung eines Immunoassays mit erfindungsgemäßen biotinylierten Bindungssubstanzen gegen Antikörper, gerichtet gegen das gp32 Epitop des HIV-Virus

In den eingesetzten Bindungssubstanzen (gp32-4 bis gp32-20) wurden gegenüber dem natürlichen Epitop (HIV gp32-Bi. "Standardsequenz") eine oder mehrere natürliche Aminosäuren durch unnatürliche Aminosäuren 1-7 ersetzt (Tabelle 5).

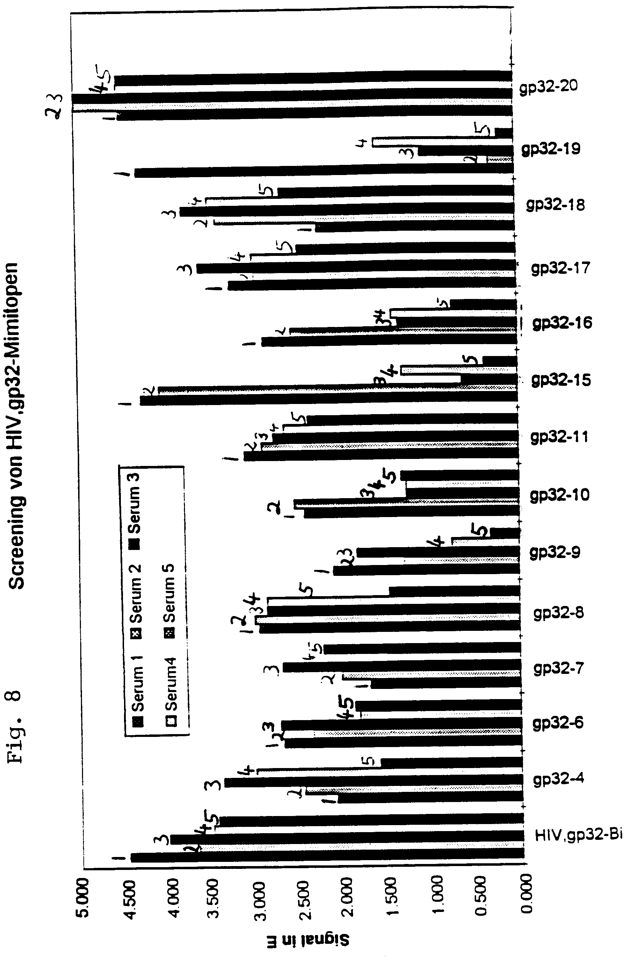

Der Immunoassay wurde analog Beispiel 1 mit 5 verschiedenen Seren durchgeführt. Figur 8 zeigt die relativen Reaktivitaten der verschiedenen Bindungssubstanzen im Vergleich zum natürlichen Epitop. Zum Teil konnte sogar eine höhere Reaktivität gemessen werden.

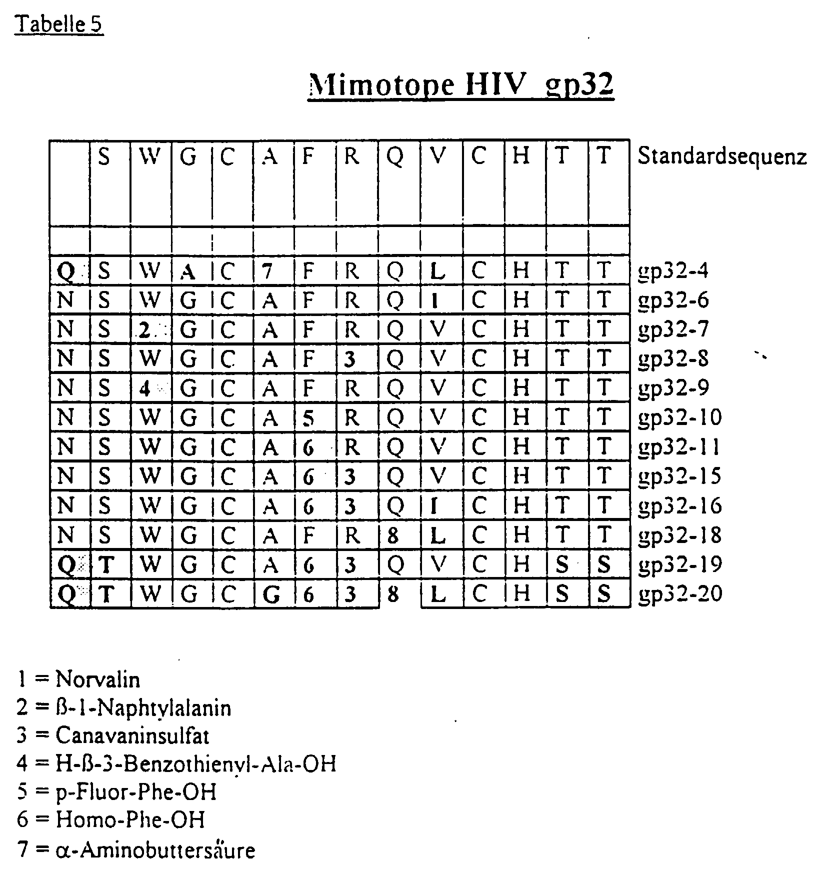

## Patentansprüche

1. Immunologische Bindungssubstanz, die an einen Antikörper, der gegen ein HCV-Epitop mit einer natürlichen Aminosäuresequenz spezifisch gerichtet ist, spezifisch bindet und die entweder eine detektierbare Markierung trägt oder festphasengebunden ist oder über ein spezifisches Bindungsmolekül an eine Festphase gebunden werden kann oder an ein immunogen wirksames, T-Zell-Epitop enthaltendes Molekül oder an ein Carrierproteinmolekül gebunden ist,
**dadurch gekennzeichnet**,
daß die spezifische Bindungssubstanz eine dem natürlichen HCV-Epitop entsprechende Aminosäuresequenz darstellt, bei der mindestens an einer Peptidbindung oder an einer Aminosäure die Sequenz verändert ist durch wenigstens eine der Modifikationen:
a) eine oder mehrere -CO-NH-Peptidbindungsgruppen sind durch eine davon verschiedene zwei- oder dreiatomige Brücke ersetzt,
b) Aminosäureseitenketten sind um eine -CH₂-Gruppe verkürzt oder um eine -CH₂-, -S-, -O-, -NH-, -SO₂- oder -CO-Gruppe verlängert, so daß keine natürlichen Aminosäurereste entstehen, oder/und
c) eine oder mehrere Aminosäuren sind durch eine nicht-biogene L-oder D-Aminosäure des Typs HR'N-Y-COOH ersetzt, wobei
R' Wasserstoff und
Y eine -(CH₂)ₙ-Gruppe darstellt, mit n = 2 - 8 oder eine -CH-CH₂)ₘ-CR¹R²R³ Gruppe darstellt mit m = 0 - 3, wobei
R¹, R² und R³ die gleich oder verschieden sein können, Wasserstoff, einen verzweigten oder unverzweigten C₁-C₄-Alkylrest darstellen oder einen Phenyl-, Naphtyl- oder einen O, S oder N enthaltenden 5-6 gliedrigen Heteroarylrest darstellen, der mit Methyl, Halogen, NH₂, OH oder Carboxy substituiert sein kann oder wobei
Y eine CHR-Gruppe darstellt, wobei
R eine Seitenkette einer natürlichen Aminosäure darstellt, in der eine -CH₂-Gruppe durch -S-, -NH-, -CH=-, -SO₂-, -CO- oder -O-ersetzt ist, oder in der ein oder mehrere H-Atome durch Methyl, NH₂, Carboxyl, SH, Halogen oder Hydroxy ersetzt sind,
wobei in allen Fällen Y eine in keiner natürlichen Aminosäure vorkommende Gruppierung bildet, oder wobei R' Methyl, Ethyl oder Phenyl und Y eine CHR-Gruppe darstellt, in der R eine Seitenkette einer natürlichen Aminosäure ist.

2. Immunologische Bindungssubstanz gemäß Anspruch 1,
**dadurch gekennzeichnet**,
daß die -CO-NH-Gruppe einer Peptidbindung durch eine -NH-CO-, -CH₂-CH₂-, -CH = CH-, -CH₂-NH-, -NH-CH₂-, -S-CH₂-, -CH₂-S-, -CH₂O-,

3. Immunologische Bindungssubstanz gemäß Anspruch 2,
**dadurch gekennzeichnet**,
daß die -CO-NH-Gruppe durch eine Gruppe, ausgewählt aus -CH₂-CH₂-,

4. Immunologische Bindungssubstanz gemäß Anspruch 1 bis 3,
**dadurch gekennzeichnet**,
daß eine -NH-CO-Gruppe an einer proteasespaltungsgefährdeten Peptidbindung ersetzt ist.

5. Immunologische Bindungssubstanz gemäß Anspruch 1 bis 4,
**dadurch gekennzeichnet**,
daß die -CO-NH-Gruppe zwischen zwei Aminosäuren, von denen mindestens eine Glycin ist, ersetzt ist.

6. Immunologische Bindungssubstanz gemäß Anspruch 1,
**dadurch gekennzeichnet**,
daß alle Aminosäureseitenketten um eine -CH₂-Gruppe verlängert sind.

7. Immunologische Bindungssubstanz gemäß Anspruch 1 bis 6,
**dadurch gekennzeichnet**,
daß die Bindungssubstanz an ein spezifisches Bindungsmolekül für eine Festphasenbindungsstelle gebunden ist.

8. Immunologische Bindungssubstanz gemäß Anspruch 7,
**dadurch gekennzeichnet**,
daß das spezifische Bindungsmittel Biotin ist.

9. Vakzin enthaltend als Immunogen eine immunologische Bindungssubstanz, die an ein Carrier-Molekül oder ein immunogen wirksames Molekül, das ein T-Zell-Epitop enthält, gekoppelt ist, wobei die Bindungssubstanz mit einem Antikörper, der gegen ein HCV-Epitop mit einer natürlichen Aminosäuresequenz gerichtet ist, spezifisch bindet und zusammen mit dem Carrier-Molekül fähig ist, solche Antikörper als Immunantwort zu erzeugen,
**dadurch gekennzeichnet**,
daß die Bindungssubstanz eine an einen immunogenen wirksamen Carrier gebundene Bindungssubstanz gemäß Anspruch 1 bis 8 ist.

10. Verfahren zur Herstellung von Antikörpern, die gegen ein HCV-Epitop mit einer natürlichen Aminosäuresequenz gerichtet sind,
**dadurch gekennzeichnet**,
daß ein Säugetier mit einem Immunogen gemäß Anspruch 9 immunisiert wird und Antikörper nach üblichen Methoden gewonnen werden.

11. Immunoassay zum Nachweis eines Antikörpers, der gegen ein HCV-Epitop mit einer natürlichen Aminosäuresequenz gerichtet ist, durch Inkubation der Antikörperprobe mit zwei Bindungspartnern, die mit dem Antikörper spezifisch binden, und Bestimmung der Bildung des Antikörper-Bindungspartner-Komplexes in geeigneter Weise,
**dadurch gekennzeichnet**,
daß als Bindungspartner für den Antikörper mindestens eine Bindungssubstanz gemäß Anspruch 1 bis 8 verwendet wird.

12. Immunologisches Bestimmungsverfahren zum Nachweis eines Antikörpers, der gegen HCV-Epitop mit einer natürlichen Aminosäuresequenz gerichtet ist, mit Hilfe einer ersten Bindungssubstanz, die festphasengebunden vorliegt oder über ein spezifisches Bindungsmolekül an eine Festphase gebunden werden kann und die spezifisch mit dem Antikörper bindet, und einem markierten Bindungspartner, der mit dem Antikörper spezifisch bindet,
durch Kontaktieren der Antikörperprobe mit der spezifischen Bindungssubstanz, dem markierten Bindungspartner und der Festphase und Messung der Markierung an der Festphase oder in freier Phase als Maß für die Menge des Analyten,
**dadurch gekennzeichnet**,
daß die spezifische Bindungssubstanz eine Bindungssubstanz gemäß Anspruch 1 bis 8 ist.

13. Immunologisches Bestimmungsverfahren gemäß Anspruch 12,
**dadurch gekennzeichnet**,
daß auch der markierte Bindungspartner eine markierte spezifische Bindungssubstanz gemäß Anspruch 1 bis 8 ist.

14. Verfahren gemäß Anspruch 12,
**dadurch gekennzeichnet**,
daß der markierte Bindungspartner ein gegen den Antikörper gerichteter Anti-Antikörper ist.

15. Verfahren gemäß Anspruch 12 bis 14,
**dadurch gekennzeichnet**,
daß der Bindungspartner enzymmarkiert ist oder durch Chemilumineszenz oder Fluoreszenz markiert ist.

16. Verwendung einer spezifischen Bindungssubstanz gemäß Anspruch 1 bis 8 für Immunoassays.

17. Verwendung einer spezifischen Bindungssubstanz gemäß Anspruch 9 zur Herstellung eines Vakzins.

18. Herstellung einer spezifischen Bindungssubstanz gemäß Anspruch 1 durch Bindung der das C-terminale Ende bildenden Aminosäure an einen Träger, schrittweisen Aufbau der Aminosäuresequenz nach bekannten Methoden, Abspaltung des C-terminalen Endes der Sequenz vom Träger und Kopplung der Sequenz vor oder nach der Abspaltung vom Träger an ein Markierungsmolekül, an ein spezifisches Festphasenbindemolekül, an eine Festphase, an ein Carriermolekül oder an ein T-Zell-Epitop enthaltendes Molekül,
**dadurch gekennzeichnet,**
daß für die Peptidsynthese an der Stelle einer proteasespaltungsgefährdeten Peptidbindung oder einer Aminosäure der natürlichen HCV-Epitopsequenz statt
a) der nacheinander dieser Peptidbindung bildenden Aminosäuren NH₂-CHR¹-COOH und NH₂-CHR²-COOH eine nicht natürliche Aminosäure NH₂-CHR¹-X-CHR²-COOH verwendet wird, in der R¹ und R², die gleich oder verschieden sein können, Seitenketten von natürlichen Aminosäuren bilden und X eine zwei- oder dreiatomige Brücke bildet,
b) einer Aminosäure des natürlichen Epitops
- eine Aminosäure verwendet wird, deren Seitenkette um eine - CH₂-Gruppe verkürzt oder um eine -CH₂-, -S-, -O-, -NH-, -SO₂-, oder -CO-Gruppe verlängert ist, so daß kein natürlicher Aminosäurerest entsteht,
- eine nicht-biogene L- oder D-Aminosäure des Typs HR'N-Y-COOH verwendet wird, in der
R' Wasserstoff und
Y eine -(CH₂)ₙ-Gruppe darstellt mit n = 2 - 8 oder
eine -CH-(CH₂)ₘ-CR¹R²R³-Gruppe darstellt mit m = 0 - 3,
wobei
R¹, R² und R³, die gleich oder verschieden sein können, Wasserstoff, einen verzweigten oder unverzweigten C₁-C₄-Alkylrest darstellen oder einen Phenyl-, Naphtyl- oder einen O, S oder N enthaltenden 5-6 gliedrigen Heteroarylrest darstellen, der mit Methyl, Halogen, NH₂, OH oder Carboxy substituiert sein kann, oder wobei
Y eine CHR-Gruppe darstellt, wobei
R eine Seitenkette einer natürlichen Aminosäure darstellt, in der eine -CH₂-Gruppe durch -S-, -NH-, -CH =-, -SO₂-, -CO- oder -O- ersetzt ist, oder in der ein oder mehrere H-Atome durch CH₃, NH₂, Carboxyl, SH-Halogen oder Hydroxy ersetzt sind, wobei in allen Fällen Y eine in keiner natürlichen Aminosäure vorkommende Gruppierung bildet, oder
wobei R' Methyl, Ethyl oder Phenyl und Y eine CHR-Gruppe darstellt, in der R eine Seitenkette einer natürlichen Aminosäure ist.

## Claims

1. Immunological binding substance which specifically binds to an antibody that is specifically directed against a HCV epitope with a natural amino acid sequence and which either carries a detectable label or is bound to a solid phase or can be bound to a solid phase via a specific binding molecule or is bound to an immunogenically active molecule containing a T cell epitope or to a carrier protein molecule,
**wherein**
the specific binding substance represents an amino acid sequence which corresponds to the natural HCV epitope in which the sequence is modified at least at one peptide bond or at an amino acid by at least one of the modifications
a) one or several -CO-NH peptide bond groups are replaced by a diatomic or triatomic bridge which differs therefrom,
b) amino acid side chains are shortened by a -CH₂ group or extended by a -CH₂, -S, O, -NH, SO₂, -CO group so that no natural amino acid residues are formed or/and
c) one or several amino acids are replaced by a non-biogenic L- or D-amino acid of the type HR'N-Y-COOH in which
R' represents hydrogen and
Y represents a -(CH₂)ₙ- group in which n = 2-8 or a -CH-(CH₂)ₘ-CR¹R²R³ group in which m = 0 - 3 and
R¹, R² and R³ can be the same or different, represent hydrogen, a branched or unbranched C₁-C₄ alkyl residue or a phenyl, napthyl or 5-6-membered heteroaryl residue containing an O, S or N which can be substituted with methyl, halogen, NH₂, OH or carboxy or
Y represents a CHR group in which
R represents a side chain of a natural amino acid in which a -CH₂- group is replaced by -S-, -NH-, -CH=, -SO₂-, -CO- or -O- or in which one or several H atoms are replaced by methyl, NH₂, carboxyl, SH, halogen or hydroxy, wherein in all cases Y forms a group which does not occur in any natural amino acid or wherein R' is methyl, ethyl or phenyl and Y represents a CHR group in which R is a side chain of a natural amino acid.

2. Immunological bindinq substance as claimed in claim 1,
**wherein**
the -CO-NH group of a peptide bond is replaced by a -NH-CO-, -CH₂-CH₂-, -CH=CH-, -CH₂-NH-, -NH-CH₂-, -S-CH₂, -CH₂-S-, -CH₂-O-, -O-CH₂- or -CH₂-CH₂-CH₂-group.

3. Immunological binding substance as claimed in claim 2,
**wherein**
the -CO-NH- group is replaced by a group selected from -CH₂-CH₂-, -CH₂-NH-, -NH-CO-.

4. Immunological binding substance as claimed in claim 1-3,
**wherein**
an -NH-CO- group is replaced at a peptide bond which is susceptible to protease cleavage.

5. Immunological binding substance as claimed in claim 1 to 4,
**wherein**
the -CO-NH- group is replaced between two amino acids one of which is at least a glycine.

6. Immunological binding substance as claimed in claim 1,
**wherein**
all amino acid side chains are extended by a -CH₂-group.

7. Immunological binding substance as claimed in claim 1 to 6,
**wherein**
the binding substance is bound to a specific binding molecule for a solid phase binding site.

8. Immunological binding substance as claimed in claim 7,
**wherein**
the specific binding molecule is biotin.

9. Vaccine containing an immunological binding substance as the immunogen which is coupled to a carrier molecule or an immunogenically active molecule containing a T cell epitope wherein the binding substance specifically binds to an antibody that is directed against a HCV epitope with a natural amino acid sequence and, together with the carrier molecule, is capable of producing such antibodies as an immune response,
**wherein**
the binding substance is a binding substance as claimed in claims 1-8 that is bound to an immunogenically active carrier.

10. Process for the production of antibodies which are directed against a HCV epitope with a natural amino acid sequence,
**wherein**
a mammal is immunized with an immunogen as claimed in claim 9 and antibodies are isolated according to the usual methods.

11. Immunoassay for the detection of an antibody which is directed against a HCV epitope with a natural amino acid sequence by incubating the antibody sample with two binding partners which specifically bind to the antibody and determining the formation of the antibody-binding partner complex in a suitable manner,
**wherein**
at least one binding substance as claimed in claims 1-8 is used as the binding partner for the antibody.

12. Immunological method of determination for the detection of an antibody which is directed against a HCV epitope with a natural amino acid sequence with the aid of a first binding substance which is present bound to a solid phase or can be bound to a solid phase via a specific binding molecule and which specifically binds to the antibody and a labelled binding partner which specifically binds to the antibody
by contacting the antibody sample with the specific binding substance, the labelled binding partner and the solid phase and measuring the label on the solid phase or in the free phase as a measure for the amount of analyte,
**wherein**
the specific binding substance is a binding substance as claimed in claims 1 to 8.

13. Immunological method of determination as claimed in claim 12,
**wherein**
the labelled binding partner is also a labelled specific binding substance as claimed in claims 1 to 8.

14. Method as claimed in claim 12,
**wherein**
the labelled binding partner is an anti-antibody which is directed against the antibody.

15. Method as claimed in claims 12 to 14,
**wherein**
the binding partner is labelled with an enzyme or has a chemiluminescent or fluorescent label.

16. Use of a specific binding substance as claimed in claims 1 to 8 for immunoassays.

17. Use of a specific binding substance as claimed in claim 9 for the production of a vaccine.

18. Production of a specific binding substance as claimed in claim 1 by binding the amino acid that forms the C-terminal end to a carrier, step-wise synthesis of the amino acid sequence according to known methods, cleaving the C-terminal end of the sequence from the carrier and coupling the sequence, before or after cleavage from the carrier, to a marker molecule, to a specific solid phase binding molecule, to a solid phase, to a carrier molecule or to a molecule containing a T cell epitope,
**wherein**
at the position of a peptide bond that is susceptible to protease cleavage or an amino acid of the natural HCV epitope sequence
a) a non-natural amino acid NH₂-CHR¹-X-CHR²-COOH is used for the peptide synthesis instead of the amino acids NH₂-CHR¹-COOH and NH₂-CHR²-COOH that consecutively form this peptide bond in which R¹ and R² which can be the same or different form side chains of natural amino acids and X forms a diatomic or triatomic bridge,
b) an amino acid is used for the peptide synthesis instead of an amino acid of the natural epitope whose side chain is shortened by a -CH₂- group or extended by a -CH₂-, -S-, -O-, -NH-, -SO₂- or -CO- group so that no natural amino acid residue is formed
or a non-biogenic L- or D-amino acid of the type HR'N-Y-COOH is used for the peptide synthesis in which
R' represents hydrogen and
Y represents a -(CH₂)ₙ- group in which n = 2-8 or a -CH-(CH₂)ₘ-CR¹R²R³ group in which m = 0 - 3 and
R¹, R² and R³ can be the same or different and represent hydrogen, a branched or unbranched C₁-C₄ alkyl residue or a phenyl, napthyl or 5-6-membered heteroaryl residue containing an O, S or N which can be substituted with methyl, halogen, NH₂, OH or carboxy or in which
Y represents a CHR group in which
R represents a side chain of a natural amino acid in which a -CH₂- group is replaced by -S-, -NH-, -CH=, -SO₂-, -CO- or -O- or in which one or several H atoms are replaced by CH₃, NH₂, carboxyl, SH, halogen or hydroxy, wherein in all cases Y forms a group which does not occur in any natural amino acid or wherein R' is methyl, ethyl or phenyl and Y represents a CHR group in which R is a side chain of a natural amino acid.

## Revendications

1. Substance de fixation immunologique qui se fixe spécifiquement sur un anticorps qui est dirigé spécifiquement contre un déterminant antigénique ou épitope HCV avec une séquence d'acides amines naturels et qui, ou bien porte un marquage détectable ou bien est fixée en phase solide ou encore peut être fixée par l'intermédiaire d'une molécule de fixation spécifique sur une phase solide ou est fixée sur une molécule contenant un épitope de cellule T, immunogène efficace ou sur une molécule de protéine porteuse,
caractérisée en ce que la substance de fixation spécifique représente une séquence d'acides amines correspondant à l'épitope HCV naturel, la séquence étant modifiée au moins sur une fixation peptide ou sur un acide aminé par au moins l'une des modifications :
a) un ou plusieurs groupes de fixation peptide -CO-NH-sont remplacés par différents ponts à deux ou trois atomes,
b) des chaînes latérales d'acides aminés sont raccourcies d'un groupe -CH₂- ou prolongées d'un groupe -CH₂-,-S-,-O-,-NH-,-SO₂- de sorte qu'il n'est produit aucun résidu d'acides aminés naturels, ou/et
c) un ou plusieurs acides aminés sont remplacés par un acide aminé D ou L non biogène du type HR'N-Y-COOH, où
R' représente l'hydrogène et,
Y représente un groupe -(CH₂)ₙ-, avec n = 2-8 ou représente un groupe -CH-(CH₂)ₘ-CR¹R²R³ avec m = 0-3, où R¹, R² et R³ qui peuvent être identiques ou différents, représentent l'hydrogène, un radical alkyle C₁-C₄ ramifié ou non ramifié ou représentent un radical hétéroaryle à 5-6 membres contenant phényle, naphtyle ou O, S ou N, pouvant être substitués par méthyle, halogène, NH₂, OH ou carboxy ou en ce que,
Y représente un groupe CHR,
R représente une chaîne latérale ou un acide aminé naturel dans le groupe -CH₂- remplacé par -S-,NH-, -CH=-,-SO₂-,-CO- ou -O-, ou dans lequel un ou plusieurs atomes H sont remplacés par méthyle NH₂, carboxyle, SH, halogène ou hydroxy, de sorte que dans tous les cas Y forme un groupement ne se produisant dans aucun acide aminé naturel, ou en ce que R' représente méthyle, éthyle ou phényle et Y un groupe CHR, dans lequel R est une chaîne latérale d'un acide miné naturel.

2. Substance de fixation immunologique selon la revendication 1, caractérisée en ce que,
le groupe -CO-NH- d'une fixation peptide est remplacé par un groupe -NH-CO-, -CH₂-CH₂-, -CH=CH-, -CH₂-NH-, -NH-CH₂-, -S-CH₂-, CH₂-S-, -CH₂-O-, -O-CH₂- ou -CH₂-CH₂-CH₂-.

3. Substance de fixation immunologique selon la revendication 2, caractérisée en ce que,
le groupe -CO-NH- est remplacé par un groupe choisi à partir de -CH₂-CH₂-, -CH₂-NH-, -NH-CO.

4. Substance de fixation immunologique selon la revendication 1 à 3, caractérisée en ce
qu'un groupe -NH-CO- est remplacé sur une fixation peptide risquant un clivage protéase.

5. Substance de fixation immunologique selon la revendication 1 à 4, caractérisée en ce que,
le groupe -CO-NH- est remplacé entre deux acides aminés dont au moins l'un d'entre eux est une glycine.

6. Substance de fixation immunologique selon la revendication 1, caractérisée en ce que,
toutes les chaînes latérales d'acides aminés sont prolongées d'un groupe -CH₂-.

7. Substance de fixation immunologique selon la revendication 1 à 6, caractérisée en ce que,
la substance de fixation est fixée sur une molécule de fixation spécifique pour un site de fixation en phase solide.

8. Substance de fixation immunologique selon la revendication 7, caractérisée en ce que,
l'agent de fixation spécifique est la biotine.

9. Vaccin contenant en tant qu'immunogène une substance de fixation immunologique qui est accouplée à une molécule porteuse ou à une molécule immunogène efficace qui contient un épitope de cellule T, la substance de fixation avec un anticorps qui est dirigée contre un épitope HCV avec une séquence d'acides aminés naturels, se fixe de façon spécifique et conjointement avec la molécule porteuse et qui est capable de produire cet anticorps en tant que réponse immune,
caractérisé en ce que la substance de fixation est une substance de fixation selon la revendication 1 à 8 fixée sur le porteur immunogène efficace.

10. Procédé pour la préparation d'anticorps qui sont dirigés contre un épitope HCV avec une séquence d'acides aminés naturels, caractérisé en ce que,
on immunise un mammifère avec un immunogène selon la revendication 9 et l'on prélève l'anticorps selon les procédés habituels.

11. Dosage immunologique pour la détection d'un anticorps qui est dirigé contre un épitope HCV avec une séquence d'acides aminés naturels, par incubation de l'échantillon sonde anticorps avec deux partenaires de liaison qui se lient de façon spécifique à l'anticorps, et détermination de la formation du complexe anticorps-partenaire de liaison de la manière appropriée, caractérisé en ce que comme partenaire de liaison pour l'anticorps on utilise au moins une substance de fixation selon la revendication 1 à 8.

12. Procédé de détermination immunologique pour détecter un anticorps qui est dirigé contre un épitope HCV avec une séquence d'acides aminés naturels, à l'aide d'une première substance de fixation, qui se présente liée en phase solide ou qui peut être liée par une molécule de fixation spécifique sur une phase solide et qui se lie de façon spécifique à l'anticorps et un partenaire de liaison marqué qui se lie de façon spécifique avec l'anticorps, par mise en contact de l'échantillon anticorps avec la substance de fixation spécifique, le partenaire de liaison marqué et la phase solide et mesure du marquage sur la phase solide ou dans la phase libre comme mesure pour la quantité d'analyte,
caractérisé en ce que la substance de fixation spécifique est une substance de fixation selon la revendication 1 à 8.

13. Procédé de détermination immunologique selon la revendication 12, caractérisée en ce que le partenaire de liaison marqué est également une substance de fixation spécifique marquée selon la revendication 1 à 8.

14. Procédé selon la revendication 12,
caractérisé en ce que le partenaire de liaison marqué est un anti-anticorps dirigé contre l'anticorps.

15. Procédé selon la revendication 12 à 14,
caractérisé en ce que le partenaire de liaison est marqué par enzyme ou par chimioluminescence ou fluorescence.

16. Utilisation d'une substance de fixation selon la revendication 1 à 8 pour des tests de dosages immunologiques.

17. Utilisation d'une substance de fixation spécifique selon la revendication 9 pour la préparation d'un vaccin.

18. Préparation d'une substance de fixation spécifique selon la revendication 1 par fixation de l'acide aminé formant l' extrémité terminaison C sur un support, constitution en pas à pas de la séquence d'acides aminés selon les procédés connus, séparation de l'extrémité terminaison C de la séquence du support et couplage de la séquence avant ou après la séparation du support sur une molécule de marquage, sur une molécule de fixation en phase solide spécifique, sur une phase solide, sur une molécule porteuse ou sur une molécule contenant l'épitope de cellules T,
caractérisée en ce que,
pour la synthèse de peptide à la place d'une fixation de peptide risquant le clivage protéase ou d'un acide aminé de la séquence épitope HCV naturelle, au lieu
a) des acides aminés formant successivement cette fixation de peptide NH₂-CHR¹-COOH et NH₂-CHR²-COOH, on utilise un acide aminé naturel NH₂-CHR¹-X-CHR²-COOH, dans lequel R¹ et R² qui peuvent être identiques ou différents forment les chaînes latérales d'acides aminés naturels et X forme un pont à deux ou trois atomes,
b) on utilise un acide aminé de l'épitope naturel
- un acide aminé dont les chaînes latérales sont raccourcies d'un groupe -CH₂- ou sont prolongées d'un groupe -CH₂-, -S-, -O-, -NH-, -SO₂-, ou -CO- de sorte qu'il n'est produit aucun résidu d'acide aminé naturel
- on utilise un acide aminé L ou D non biogène du type HR'N-Y-COOH dans lequel
R' est l'hydrogène et
Y représente un groupe -(CH₂)ₙ- avec n = 2-8 ou un groupe -CH-(CH₂)ₘ-CR¹R²R³ avec m = 0-3,
dans lequel
R¹, R² et R³ qui peuvent être identiques ou différents représentent l'hydrogène, un radical alkyle C₁-C₄ linéaire ou non linéaire ou représente un radical hétéroaryle à 5 ou 6 cycles contenant phényle, naphtyle ou O, S ou N qui peut être substitué par méthyle, halogène, NH₂, OH ou carboxy ou dans lequel
Y représente un groupe CHR-,
dans lequel
R représente une chaîne latérale d'un acide aminé naturel dans lequel le groupe -CH₂- est remplacé par -S-,-NH-,-CH =-, -SO₂-,-CO- ou -O- ou dans lequel un ou plusieurs atomes H sont remplacés par CH₃, NH₂, carboxyle, SH-halogène ou hydroxy, dans lequel dans tous les cas Y forme un groupement ne se présentant dans aucun acide aminé naturel ou dans lequel R' représente méthyle, éthyle ou phényle et Y un groupe CHR, dans lequel R est une chaîne latérale d'un acide aminé naturel.
